(19) Europäisches Patentamt
European Patent Office
Office européen des brevets

(11) Veröffentlichungsnummer: **0 406 685 A1**

# (12) EUROPÄISCHE PATENTANMELDUNG

(21) Anmeldenummer: **90112230.9**

(22) Anmeldetag: **27.06.90**

(51) Int. Cl.5: **C08B 37/00**, C08B 37/02, C08B 37/14, A61K 31/72, A61K 31/725

(30) Priorität: **01.07.89 DE 3921761**

(43) Veröffentlichungstag der Anmeldung: **09.01.91 Patentblatt 91/02**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Magerstädt, Michael, Dr.**
**Rheingaustrasse 19**
**D-6238 Hofheim am Taunus(DE)**
Erfinder: **Schlingmann, Merten, Prof. Dr.**
**Schneidhainer Strasse 32a**
**D-6240 Königstein/Taunus(DE)**
Erfinder: **Schrinner, Elmar, Dr.**
**Hedwigstrasse 2**
**D-6100 Wiesbaden(DE)**
Erfinder: **Walch, Axel, Dr.**
**Hans-Sachs-Strasse 5**
**D-6000 Frankfurt am Main(DE)**
Erfinder: **Wiesner, Matthias, Dr.**
**Im Münchfeld 8**
**D-6500 Mainz(DE)**
Erfinder: **Winkler, Irvin, Dr.**
**In den Eichen 40**
**D-6237 Liederbach(DE)**
Erfinder: **Bader, Hubert, Dr.**
**4039 Carmel Forest Drive**
**Charlotte, NC 28226(US)**
Erfinder: **Paessens, Arnold, Dr.**
**Stresemannstrasse 56**
**D-5657 Haan(DE)**
Erfinder: **Biesert, Lothar, Dr.**
**Eschersheimer Landstrasse 576**
**D-6000 Frankfurt am Main(DE)**

## (54) Substituierte Polysaccharide, Verfahren zu deren Herstellung sowie deren Verwendung zur Prophylaxe und Behandlung von Viruserkrankungen.

(57) Polysaccharide, die zu einem gewissen Grad sulfatiert sind und weiterhin substituiert sind mit einer Gruppe der Formel -X-B-Y, wobei dieser Substituent die genannten Bedeutungen hat, eignen sich zur Prophylaxe und Behandlung von Viruserkrankungen, insbesondere von Erkrankungen, die durch das HIV hervorgerufen sind.

EP 0 406 685 A1

## SUBSTITUIERTE POLYSACCHARIDE, VERFAHREN ZU DEREN HERSTELLUNG SOWIE DEREN VERWENDUNG ZUR PROPHYLAXE UND BEHANDLUNG VON VIRUSERKRANKUNGEN

Die vorliegende Erfindung betrifft substituierte Polysaccharide, Verfahren zu deren Herstellung sowie deren Verwendung zur Prophylaxe und Behandlung von Viruserkrankungen. Es ist bereits bekannt, daß sich sulfatierte Polysaccharide zur Behandlung von Viruserkrankungen eignen (vgl. z.B. WO 88/05 301, EP 0240098). Obwohl diese Verbindungen eine Wirksamkeit aufweisen, sind sie z.B. mit dem Nachteil behaftet, daß sie die Blutgerinnung beeinflussen ( vgl. z.B. Ryde et al. in Thrombosis Research 23 (1981) 435-445). In der Absicht, bessere Verbindungen zur Behandlung von Viruserkrankungen zu erhalten, wurde nun überraschenderweise gefunden, daß Polysaccharide, deren OH-Gruppen nur teilweise sulfatiert sind und ein weiterer Teil der OH-Gruppen verethert oder verestert ist, eine größere Wirksamkeit gegen Viren aufweisen als nur sulfatierte polysaccharide und gleichzeitig die Blutgerinnung weniger beeinflussen. Einige der erfindungsgemäß verwendbaren Verbindungen sind bereits aus der japanischen Patentanmeldung Sho-46-36277 bekannt, in der allerdings nur ihre Wirksamkeit für völlig andere Applikationen beschrieben wird. Die Wirksamkeit der genannten Verbindungen gegen Viren war angesichts der bereits bekannten Wirkungen der Verbindungen - wie z.B. Erweiterung der periphären Gefäße und Verbesserung des Blutkreislaufs - nicht zu erwarten.

Die beschriebene Wirksamkeit der erfindungsgemäßen Verbindungen ist auch überraschend, weil z.B. verschiedenartigste neutrale Polysaccharide wie Xylofuran, Ribofuran sowie verschiedene Dextrane keinerlei Anti-HIV-Wirksamkeit zeigen (H. Nakashima et al., Jap. J. Cancer Res./Gaun 78 (11), 1164-68, 1987) und weil viele von deren kationischen und neutralen Derivaten in gleicher Weise unwirksam sind (P. Ebbesen, Brit. J. Cancer, 30 (1), 68-72, 1974).

Darüber hinaus ist das Auffinden von pharmazeutisch wirksamen polymeren Verbindungen besonders schwierig, weil die pharmazeutische Wirksamkeit von niedermolekularen Verbindungen in keiner Weise Rückschlüsse über die pharmazeutische Wirksamkeit von Polymeren zuläßt. So ist z.B. bekannt, daß einige niedermolekulare Wirkstoffe hinsichtlich ihrer Zellmembrangängigkeit durch Einführung lipophiler Gruppen verändert werden, wohingegen bei hochmolekularen Substanzen eine derartige Veränderung bei Einführung lipophiler Gruppen nicht auftritt (Advanced Drug Delivery Reviews 1, 1987, 235-248, Elsevier).

Erfindungsgegenstand sind demzufolge substituierte Polysaccharide, die linear oder verzweigt sein können, bestehend aus einheitlichen oder unterschiedlichen natürlichen oder synthetischen Monomeren, die pro Monomereinheit auch eine substituierte oder unsubstituierte Aminogruppe enthalten können und deren OH-Gruppen durchschnittlich zwischen 5 und 80 % substituiert sind mit einer Gruppe der Formel -X-B-Y, wobei

X eine Oxygruppe oder eine Gruppe der Formel I bedeutet

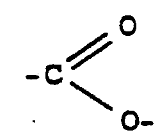

(I)

deren Kohlenstoffatom an B gebunden ist,

B einen nicht-aromatischen Kohlenwasserstoffrest mit 2 bis 30 C-Atomen bedeutet, in dessen Alkylkette bis zu 3 Methyleneinheiten durch Oxygruppen ersetzt sein können, in der bis zu drei C-C-Doppelbindungen vorliegen können und der substituiert sein kann mit bis zu drei $C_1$-$C_4$-Alkylresten und

Y - falls X eine Oxygruppe ist - Wasserstoff, COOR oder $OSO_3R^1$, sein kann, worin R ein physiologisch verträgliches ein- oder zweiwertiges Kation ist, oder einen Kohlenwasserstoffrest mit bis zu 20 C-Atomen oder einen Mono- oder Bisetherrest mit 3 bis 10 C-Atomen darstellt,

$R^1$ ein physiologisch verträgliches Kation darstellt, oder

Y - falls X ein Rest der Formel I ist - Wasserstoff oder COOR darstellt, worin R die obengenannten Bedeutungen hat

und wobei die OH-Gruppen der obengenannten Polysaccharide zwischen 10 und 95 % substituiert sind mit einer Gruppe der Formel $OSO_3M$ wobei M ein physiologisch verträgliches Kation darstellt und zwischen 0 und 40 % der OH-Gruppen unsubstituiert sind mit der Ausnahme der Palmitinsäureester von Chondroitinsulfat und Laurinsäureester von Dextransulfat, die bereits in der obengenannten japanischen Schrift Sho-46-36277 genannt sind.

Bevorzugt sind substituierte Polysaccharide, die ein Polysaccharidgerüst besitzen, das aus einheitlichen

oder verschiedenen Monomereinheiten, ausgewählt aus der folgenden Gruppe von Verbindungen aufgebaut ist bzw. aus folgenden Verbindungen besteht: Xylose, Arabinose, Rhamnose, Fucose, Glucose, Mannose, Galactose, Fructose, Glucosamin, Galactosamin, Mannosamin, Glucuronsäure, Galacturonsäure, Mannuronsäure, Carragenane, Fucoidan, Laminaran, Alginat, Lentinan, Pluran, Xylan, Dextran, Heparin, Keratansulfat, Chondroitin-4- und -6-sulfat, Dermatansulfat, Heparinsulfat, Hyaluronsäure, Teichuronsäure sowie Teilhydrolysate von Stärke, Cellulose, Glycogen, Chitin oder Pektin. Die genannten Verbindungen können sowohl synthetischer als auch natürlicher, d.h. insbesondere pflanzlicher oder mikrobieller Herkunft sein.

Die Verbindungen, die bereits in der Natur in sulfatierter Form auftreten, können sowohl mit ihrem natürlichen Sulfatgehalt als auch nach zusätzlicher Sulfatierung verwendet werden. Besonders bevorzugte Polysaccharide sind Xylan und Dextran.

Die OH-Gruppen der erfindungsgemäßen Polysaccharide sind vorzugsweise zu 5 bis 80 %, besonders bevorzugt zu 5 bis 50 % und insbesondere zu 10 bis 40 % substituiert mit einer Gruppe der Formel -X-B-Y und weiterhin vorzugsweise zu 10 bis 95 %, besonders bevorzugt zu 45 bis 95 % und insbesondere zu 50 bis 90 % substituiert mit einer Gruppe der Formel $OSO_3M$, wobei vorzugsweise zwischen 0 und 40 %, besonders bevorzugt zwischen 0 und 20 % und insbesondere zwischen 0 und 10 % der OH-Gruppen unsubstituiert sind.

Bevorzugte Reste B weisen 2 bis 20 C-Atome auf, haben eine oder keine Doppelbindung und sind nicht oder einfach substituiert mit einer $C_1$-$C_4$-Alkylgruppe.

Sofern X eine Oxygruppe ist, ist Y vorzugsweise H, COOR, $OSO_3R^1$, wobei R ein Alkalikation, insbesondere Kalium oder Natrium oder eine verzweigte oder unverzweigte $C_1$-$C_8$-Alkylgruppe ist, $R^1$ ist vorzugsweise ein Alkalikation, insbesondere Natrium.

Falls X ein Rest der Formel I ist, bedeutet Y vorzugsweise H oder einen Rest der Formel COOR, wobei R ein Alkalikation, vorzugsweise Natrium oder Kalium, eine verzweigte oder unverzweigte $C_1$-$C_8$-Alkylgruppe oder ein Mono- oder Bisetherrest mit 6 bis 9 C-Atomen ist.

Beispielhafte Reste der Formel -X-B-Y sind Ethyloxy, Propyloxy, Isopropyloxy, Butyloxy, Hexyloxy, Octyloxy, Decyloxy, Dodecyloxy, Propionyloxy, Butyryloxy, Valeryloxy, Hexanoyloxy, Octanoyloxy, Decanoyloxy, Dodecanoyloxy, Palmitoyloxy, Stearoyloxy, 2-Methylbutyryloxy, 9-Octadecenoyloxy, Linoloyloxy, Linolenoyloxy, Cholesteryloxy, Hydroxyoxalyloxy, Hydroxymalonyloxy, Hydroxysuccinyloxy, Hydroxyglutaryloxy, Hydroxyadipoyloxy, Crotonoyloxy, Mesaconoyloxy bzw. die Ester der genannten Dicarbonsäurederivate mit unverzweigten $C_1$-$C_8$-Alkoholen oder [(1,3-Diisopropoxy)-propyl-2-oxycarbonyl]-propanoyloxy.

Besondere Bedeutung besitzen die Dodecanoyloxy- und die Stearoyloxyreste, insbesondere an einem Xylan-Polysaccharidgerüst.

Die erfindungsgemäßen substituierten Polysaccharide können ein mittleres Molekulargewicht aufweisen, das einen weiten Bereich überstreicht. Vorzugsweise haben die substituierten Polysaccharide ein mittleres Molekulargewicht von bis zu 100 kD, besonders bevorzugt von bis zu 40 kD, insbesondere zwischen 1 und 22 kD. Eine besondere Bedeutung haben erfindungsgemäße substituierte Polysaccharide, deren Polysaccharidgerüst aus Xylan oder Dextran besteht und die ein mittleres Molekulargewicht von 1 bis 22 kD aufweisen.

Weiterhin gehört zu der vorliegenden Erfindung ein Verfahren zur Herstellung von substituierten Polysacchariden, in denen X eine Oxygruppe ist und das dadurch gekennzeichnet ist, daß die Polysaccharide unter basischer Katalyse mit einem Alkylhalogenid oder einem $\omega$-Halogencarbonsäuresalz partiell verethert und anschließend direkt oder nach Alkoxylierung ganz oder teilweise sulfatiert werden. Bei diesem Verfahren findet die Alkylierung z.B. mit einem Alkylhalogenid bzw. $\omega$-Halogencarbonsäuresalz in einem wasserfreien Lösungsmittel wie z.B. DMF, Dioxan, DMSO etc. statt, wobei als basischer Katalysator z.B. ein Alkalihydroxid herangezogen werden kann. Als Alkylhalogenide eignen sich insbesondere die Alkylbromide und -jodide. Eine besonders bevorzugte Variante dieses Verfahrens ist in der allgemeinen Arbeitsvorschrift I beschrieben.

Weiterhin gehört zu der vorliegenden Erfindung ein Verfahren zur Herstellung der substituierten Polysaccharide, in denen X eine Gruppe der Formel I ist und das dadurch gekennzeichnet ist, daß die Polysaccharide mit einer Mono-oder Dicarbonsäure oder mit einem Dicarbonsäuremonoester partiell acyliert werden und die verbleibenden OH-Gruppen der Polysaccharide ganz oder teilweise sulfatiert werden. Zur Acylierung können unterschiedliche Methoden benutzt werden (vgl. z.B. Organikum, VEB, Verlag der Wissenschaften, Berlin 1977, Kapitel 7 oder Houben-Weyl, Bd. 8, S. 503, Georg-Thieme-Verlag Stuttgart (1952)).

Ein bevorzugtes Verfahren besteht darin, daß man eine Lösung der in das Polysaccharid einzuführenden Carbonsäure bzw. deren Derivat in einem organischen Lösungsmittel, wie z.B. wasserfreiem DMF oder DMSO bei einer Temperatur von ca. 20 bis 60° C mit einer äquimolaren Menge N,N'-Carbonyldiimidazol umsetzt und mit dem gebildeten Acylimidazol das jeweilige Polysaccharid acyliert, was bei ca. 20 bis 60° C

durch Zufügen des jeweiligen Polysaccharids in den bereits genannten Lösungsmitteln geschehen kann. Der Acylierungsgrad wird dabei bestimmt von dem Mengenverhältnis des Acylimidazols zu dem jeweiligen Polysaccharid. Ein bevorzugtes Verfahren zur Acylierung der Polysaccharide ist in der allgemeinen Arbeitsvorschrift II beschrieben.

Die Sulfatierung der substituierten Polysaccharide kann auf unterschiedliche Weise geschehen (vgl. z.B. Whistler et al. in Carbohydr. Chem. 2, 298 (1963), US 2 715 091 oder CH 293 566). Ein bevorzugtes Verfahren besteht darin, daß man das zu sulfatierende Polysaccharid in einem organischen Lösungsmittel, vorzugsweise DMF aufnimmt und anschließend mit komplexiertem Schwefeltrioxid, vorzugsweise komplexiert mit Pyridin, bei einer Temperatur von ca. 30 bis 60° C versetzt. Erfahrungsgemäß sind zur Sulfatierung einer OH-Gruppe ca. 1,5 bis 3 Äquivalente an Schwefeltrioxid-Komplex nötig. Nach erfolgter Sulfatierung kann das Reaktionsprodukt in Wasser aufgenommen, vorzugsweise mit NaOH neutralisiert werden und anschließend nach bekannten Methoden aufgearbeitet werden. Ein besonders bevorzugtes Verfahren zur Sulfatierung der erfindungsgemäßen Polysaccharide ist in der allgemeinen Arbeitsvorschrift III beschrieben.

Polysaccharide, in denen eine Aminofunktion vorhanden ist, sollten vor der Sulfatierung mit einer Schutzgruppe versehen werden, die nach erfolgter Reaktion wieder abgespalten werden kann. Geeignete Schutzgruppen sind z.B. von E.Wünsch in Houben-Weyl, Bd. 15, I/II, Georg Thieme Verlag Stuttgart (1974) beschrieben.

Die vorliegende Erfindung betrifft weiterhin die Verwendung von substituierten Polysacchariden zur Bekämpfung von Krankheiten, die durch Viren hervorgerufen werden. Die substituierten Polysaccharide sind wirksam gegen eine Reihe von humanpathogenen Viren, wie z.B. Herpesviren; besondere Bedeutung besitzt jedoch ihre Wirksamkeit gegen Retroviren, insbesondere gegen das HIV. Zur Bekämpfung, d.h. zur Behandlung bzw. Prophylaxe von Erkrankungen, die durch Viren - insbesondere durch Retroviren - verursacht werden, können die erfindungsgemäßen Verbindungen auf unterschiedliche Weise verabreicht werden. Zum Beispiel können sie intravenös, intramuskulär, intraperitoneal, subkutan, als Dauertropfinfusion, rektal oder oral verabreicht werden. Bei akuten Krankheitszuständen ist die Verabreichung als Dauertropfinfusion vorzuziehen. Zur Dauermedikation ist z.B. die orale Verabreichung angezeigt.

Weiterhin betrifft die vorliegende Erfindung Arzneimittel, die durch einen Gehalt an erfindungsgemäßen substituierten Polysacchariden gekennzeichnet sind. Die Herstellung der erfindungsgemäßen Arzneimittel erfolgt, indem mindestens ein substituiertes Polysaccharid gegebenenfalls mit weiteren Hilfs- und/oder Trägerstoffen in eine geeignete Darreichungsform gebracht wird. Die Hilfs- und Trägerstoffe stammen aus der Gruppe der Trägermittel, Konservierungsstoffe und anderer üblicher Hilfsstoffe.

Zum Beispiel können für orale Darreichungsformen Hilfsstoffe wie Stärken, z.B. Kartoffel-, Mais- oder Weizenstärke, Cellulose bzw. deren Derivate, insbesondere mikrokristalline Cellulose, Siliziumdioxid, verschiedene Zucker wie Milchzucker, Magnesiumcarbonat und/oder Calciumphosphate verwendet werden. Weiterhin ist es vorteilhaft, den oralen Darreichungsformen Hilfsstoffe zuzusetzen, die die Verträglichkeit der Medikamente verbessern, wie z.B. Schleimbildner und Harze. Zwecks besserer Verträglichkeit können die Medikamente auch in Form von magensaftunlöslichen Kapseln verabreicht werden. Darüber hinaus kann es vorteilhaft sein, der Darreichungsform, bzw. einer Komponente des Kombinationspräparates, ein Retardierungsmittel zuzusetzen, gegebenenfalls in Form von permeablen Membranen, wie z.B. solche auf Cellulose- oder Polystyrolharzbasis oder Ionenaustauscher.

Die anzuwendende Dosierung der erfindungsgemäßen Arzneimittel ist abhängig von verschiedenen Faktoren wie Darreichungsform des Medikamentes und Zustand, Gewicht und Art der Erkrankung des Patienten. Eine Tagesdosis von ca. 5000 mg eines substituierten Polysaccharids sollte jedoch nur kurzzeitig überschritten werden. Bevorzugt sind ca. 10 bis 2500 mg substituiertes Polysaccharid als Tagesdosis für einen Menschen von ca. 70 kg Körpergewicht. Die Verabreichung der Tagesdosis der substituierten Polysaccharide kann in Form einer einzelnen Verabreichung oder in mehreren kleinen Dosen erfolgen. Bevorzugt ist die Verabreichung in 3 bis 8 Dosen pro Tag. In manchen Fällen kann eine kontinuierliche Zuführung der substituierten Polysaccharide z.B. in Form einer Dauertropfinfusion angezeigt sein.

Die antiviralen Eigenschaften der erfindungsgemäßen substituierten Polysaccharide in vitro und in vivo werden in folgenden Versuchen nachgewiesen:

**1. Wirksamkeit gegen HIV, Prüfung in der Zellkultur**

Methodenbeschreibung:

Medium RMPI pH 6,8

Komplettes Medium enthält zusätzlich 20 % foetales Kälberserum und 20 IU/ml rekombinantes Interleukin 2.

Zellen

Aus frischem Spenderblut mittels (R)Ficoll-Gradientenzentrifugation isolierte Lymphozyten werden unter Zusatz von 2 µg/ml Phytohämagglutinin (Wellcome) in komplettem Medium 36 Stunden bei 37° C unter 5 % $CO_2$ kultiviert. Die Zellen werden nach Zusatz von 10 % DMSO bei einer Zelldichte von 5 x $10^6$ eingefroren und in flüssigem Stickstoff gelagert. Für den Versuch werden die Zellen aufgetaut, in RMPI-Medium gewaschen und in komplettem Medium 3 - 4 Tage kultiviert.

Ansatz

Die Prüfpräparate werden in komplettem Medium gelöst, üblicherweise in einer Konzentration von 1,2 mg/ml. In 24er Multiwell-Schalen werden jeweils 0,5 ml komplettes Medium gegeben. Nach Zusatz von 0,1 ml des gelösten Präparates wird durch Übertragung von jeweils 0,1 ml das Präparat in einer geometrischen Verdünnungsreihe, Faktor 5, verdünnt. Zu allen Ansätzen werden 0,4 ml einer Suspension mit 5 x $10^5$ Lymphozyten pro ml gegeben.

Die Ansätze werden mit 0,1 ml eines Überstandes von HIV I (D 34, Georg Speyer Haus, Frankfurt/Main) oder HIV II (ROD) infizierten Lymphozyten versetzt, der so eingestellt ist, daß im Ansatz der cytopathogene Effekt nach 3 bis 4 Tagen erkennbar ist. Die Multiplizität der infektösen Einheit beträgt hierbei 0,01 - 0,02.

Der Versuchsansatz enthält damit folgende Komponenten in komplettem Medium:

| | |
|---|---|
| 0,5 ml | Präparat 200 µg/ml bis 1,6 µg/ml |
| 0,4 ml | Zellen ca. 1 x $10^6$/ml |
| 0,1 ml | HIV (1:20 - 1:50 verdünnter Zellkulturüberstand von HIV-infizierten Lymphozyten) |
| 1,0 ml | |

Die Versuchsansätze enthalten außerdem präparatfreie Infektionskontrollen bzw. Standardpräparate, z.B. 3′-Azido-3′-deoxythymidin 0,2 µg/ml oder Pentosanpolysulfat 25 µg/ml.

Auswertung

Die Auswertung erfolgt durch mikroskopische Betrachtung der Ansätze am 4. Tag nach Infektion. Anhand der virusbedingten Syncytienbildung wird die Virusvermehrung und die minimale Hemmkonzentration (MHK) bestimmt. Die Ergebnisse dieses Tests zeigt Tabelle 1.

Tabelle 1

| Aktivität gegen HIV-I in der Lymphozytenkultur | |
|---|---|
| Präparat | MHK ($\mu$g/ml) |
| Verbindung nach Beispiel 1 | 4 |
| 2 | 4 |
| 3 | 20 |
| 4 | 20 |
| 5 | 20 |
| 21 | 20 |
| 22 | < 0.8 |
| 23 | 4 |
| 24 | 20 |
| 25 | < 0.8 |
| Vergleich: Dextransulfat | |
| aus Dextran 10.000 D | 50 |
| Xylansulfat | 20 |

Tabelle 2

| Aktivität gegen HIV-II (ROD) in der Lymphozytenkultur | |
|---|---|
| Präparat | MHK ($\mu$g/ml) |
| Verbindung nach Beispiel 6 | 25 |
| 7 | 25 |
| 10 | 25 |
| 11 | 25 |
| 12 | 50 |
| Vergleich: Dextransulfat | |
| aus Dextran 1.000 D | 75 |
| aus Dextran 5.000 D | 50 |
| aus Dextran 10.000 D | 100 |

Reverse Transkriptase Hemmtest mit rekombinanter HIV-RT

Der Test wird in 96-Napf-Mikrotiterplatten durchgeführt. Ein 100 $\mu$l Ansatz enthält 100 mM Tris-HCl, pH 8,0, 80 mM KCl, 10 mM $MgCl_2$, 5 mM Dithiothreitol, 10 $\mu$l ($^3$H)dTTP (Desoxythymidintriphosphat) (121 Ci/mmol, 1,0 mCi/ml), 1,5 $\mu$g poly(rA)oligo(dT) 12-18 (Deutsche Pharmacia GmbH, Freiburg BRD) und 10 $\mu$l HIV-RT (1:200 verdünnt, DEAE/PC/Aga(rC)-Fraktion) (Hansen, J., Schulze, T., Mölling, K.: RNase H activity associated with bacterial expressed reverse transcriptase of Human T-cell Lymphotropic Virus III/Lymphadenopathy-associated Virus. J. Biol. Chem., 262, 12393 - 12396 (1987)).

Nach 90 Min. Inkubation bei 41 °C wird die markierte Nukleinsäure durch Einfrieren (-80 °C) und Wiederauftauen in Gegenwart von 50 $\mu$l 10 % TCA (Trichloressigsäure) und 50 $\mu$l Na-Pyrophosphat gefällt. Nach Aufsaugen des Ansatzes auf (R)Whatman GF/C-Filter wird mit je ca. 2 ml 10 % TCA/10 mM Na-Pyrophosphat (1:1), dann mit 10 mM Na-Pyrophosphat, mit $H_2O$ und mit 70 % Ethanol gewaschen und anschließend die gebundene Radioaktivität nach Trocknen des Filters bei 120 °C bestimmt.

In die DNA eingebaute $^3H$ cpm in Abwesenheit des Inhibitors werden als 100 % definiert. Dies entspricht einem Einbau von 80.000 bis 200.000 cpm pro Reaktion. Im Test werden unterschiedliche Konzentrationen eines Hemmstoffs untersucht. Angegeben werden die Endkonzentrationen im Ansatz in µg/ml.

Tabelle 3

| HIV-Reverse Transkriptase Hemmtest | |
|---|---|
| Präparat | IC 50 (µg/ml) |
| Verbindung nach Beispiel 9 | 0,159 |
| 13 | 0,041 |
| Vergleich: Xylansulfat | 0,497 |

Antivirale Aktivität gegen Herpes in der Zellkultur

Die Prüfsubstanzen werden in Zellkulturmedium (Dulbecco's MEM) gelöst und in einer geometrischen Verdünnungsreihe, Faktor 3, in Standard-Mikrotiterplatten in 100 µl Zellkulturmedium vorgelegt. Anschließend erfolgt die Zugabe von 100 µl einer Suspension von Hela- bzw. Vero-Zellen in Medium mit 5 % foetalem Kälberserum in einer Zelldichte von 2 x $10^5$ Zellen/ml. Die Ansätze werden mit 50 µl einer Suspension des jeweiligen Test-Virus infiziert, die so eingestellt ist, daß die Zellen innerhalb von 72 Stunden einen cytopathogenen Effekt (CPE) zeigen. Die Auswertung erfolgt durch mikroskopische Begutachtung des Zellrasens und photometrische Messung der Neutralrotaufnahme (Farbtest nach Filter). Als MHK wird die Konzentration des Präparats angenommen (µg/ml), bei der etwa 50 % der Zellen die Infektion überleben.

Tabelle 4

| Wirkung gegen Herpes | |
|---|---|
| Präparat | MHK (µg/ml) |
| Verbindung nach Beispiel 2 | 4,94 |
| 4 | 4,94 |
| 6 | 4,94 |
| 7 | 4,94 |
| 10 | 4,94 |
| 11 | <0,18 |
| 12 | 1,65 |
| 13 | 4,94 |
| 24 | 4,94 |
| 26 | 1,65 |
| 27 | 1,65 |
| Vergleich: Xylansulfat | 4,94 |

**2. In vivo Versuche in der Maus mit Retroviren**

Da für die HIV-Infektion des Menschen kein überzeugendes Infektionsmodell bei Labortieren existiert, muß bei der Prüfung von Chemotherapeutika auf Infektionen mit anderen Retroviren zurückgegriffen werden. Im vorliegenden Falle wurde die Infektion der Maus mit dem Friend Leukämie Virus gewählt.

Normale Labormäuse (NMRI = Naval Medical Research Institute) wurden durch intravenöse Injektion mit Friend-Virus enthaltendem Mäuseserum infiziert. Bei den unbehandelten Kontrolltieren entwickelt sich als Symptom der Infektion innerhalb von 2 Wochen bzw. 3 Wochen eine deutliche Vergrößerung von Milz und Leber. Die Behandlung erfolgt über 10 Tage bei intraperitonealer Applikation und über 16 Tage bei oraler Applikation der Testsubstanzen. Bei intraperitonealer Gabe der Testsubstanzen beginnt die Behandlung 48 Stunden nach der Infektion, bei peroraler Gabe der Testsubstanz 4 Tage vor der Infektion. Am 14. Tag nach Infektion werden die Tiere durch Luxation der Halswirbel getötet und geöffnet. Die Milz wird entnommen und gewogen. Als Meßparameter der therapeutischen Wirksamkeit wird das Milzgewicht der behandelten Tiere mit den der unbehandelten Infektionskontrolle in Relation gesetzt.

Für die orale Applikation werden die Prüfsubstanzen in Trinkwasser gelöst. Alle 24 Stunden werden die Trinkwasserbehälter mit jeweils 100 ml Inhalt gewechselt. Aus dem täglich ermittelten Verbrauch an Trinkwasser pro Versuchskollektiv wird die durchschnittliche Dosismenge errechnet, die einer Maus pro 24 Stunden appliziert wurde.

Während bei uninfizierten, ausgewachsenen Labormäusen (20 - 24 g Körpergewicht) die Milz weniger als 1 % des Körpergewichtes wiegt, erreicht bei infizierten Tieren die Milz am Ende des Versuchs etwa bis zu 10 % des Körpergewichtes.

Als Vergleichspräparate dienen Suramin (= 3,3′-Ureylen-bis-(8-(3-benzamido-4-methylbenzamido)-naphthalin-1,3,5-trisulfonsäure|) bei intraperitonealer Gabe bzw. Pentosanpolysulfat bei intraperitonealer und oraler Gabe.

Die Ergebnisse sind in Tabelle 5 und 6 dargestellt.

Tabelle 5

FLV-Modell der Maus

Behandlungsbeginn: 2 Tage nach Infektion
Behandlungsdauer: 10 Tage
Virusverdünnung: 1:5.000
Applikationsart: i.p.
Milzentnahme: 14 Tage nach Infektion

| Präparat Verbindung nach | Dosierung mg/Maus | rel.Milzgewicht in % Körpergew. | | Anzahl überlebender Mäuse n | Signifikanz (p) |
|---|---|---|---|---|---|
| Kontrolle | - | 6,41 | 3,11 | 10 | 1,0000 |
| Suramin | 10 x 1,0 | 4,96 | 1,45 | 10 | 0,0985 |
| XS | 10 x 0,5 | 5,67 | 2,31 | 9 | 0,2883 |
| XS | 10 x 1,0 | 5,07 | 3,19 | 6 | 0,2141 |
| Bsp. 14 | 10 x 0,1 | 3,79 | 1,50 | 6 | 0,0372 |
| Bsp. 13 | 10 x 1,0 | 2,95 | 0,95 | 5 | 0,0156 |
| Bsp. 15 | 10 x 1,0 | 2,42 | 1,00 | 6 | 0,0046 |
| Kontrolle | - | 7,25 | 2,91 | 10 | 1,0000 |
| Suramin | 10 x 0,1 | 5,68 | 2,57 | 10 | 0,1069 |
| XS | 10 x 0,5 | 5,40 | 1,53 | 8 | 0,0601 |
| XS | 10 x 1,0 | 5,60 | 2,62 | 5 | 0,1532 |
| Bsp. 16 | 10 x 1,0 | 4,94 | 1,81 | 10 | 0,0223 |
| Bsp. 11 | 10 x 1,0 | 4,60 | 1,83 | 9 | 0,0152 |
| Bsp. 17 | 10 x 0,5 | 3,79 | 1,92 | 9 | 0,0038 |

XS: Xylansulfat

Tabelle 6

| FLV-Modell der Maus | | | | | |
|---|---|---|---|---|---|
| Behandlungsbeginn: 4 Tage vor Infektion<br>Behandlungsdauer: 16 Tage<br>Virusverdünnung: 1:5.000<br>Applikationsart: oral über das Trinkwasser<br>Milzentnahme: 14 Tage nach Infektion | | | | | |
| Präparat Verbindung nach | Dosierung mg/Maus | rel.Milzgewicht in % Körpergew. | | Anzahl überlebender Mäuse n | Signifikanz (p) |
| Kontrolle | - | 7,34 | 2,36 | 9 | 1,000 |
| XS | 16 x 49,0 | 6,43 | 3,25 | 10 | 0,2548 |
| Bsp. 18 | 16 x 42,4 | 4,54 | 1,11 | 10 | 0,0019 |
| Bsp. 19 | 16 x 22,3 | 3,58 | 2,05 | 10 | 0,0010 |
| Bsp. 20 | 16 x 41,4 | 5,91 | 1,94 | 10 | 0,0835 |
| XS: Xylansulfat | | | | | |

**3. Wirkung gegen VISNA-Virus in der Zellkultur**

Das VISNA-Virus und das HIV-Virus (Virus der humanen Immundefiziens) gehören beide zu der Retrovirus-Subfamilie der Lentiviren (Haase A.T. Nature (1986) 322, 130-136). Beide Viren weisen eine ähnliche Genomorganisation und ein gegenüber den anderen Retroviren komplexes Transkriptionsmuster auf (Sonijo P. et al., Cell (1985), 42 , 369 - 382; Davis J. L. et al., Journal of Virology (1987) 61 (5) 1325-1331).

Der VISNA-Virus-Test wird nach der Methode von O. Narayan et al., Journal of Infectious Diseases 135 , 5, 1977, 800-806 durchgeführt. Dazu werden die erfindungsgemäßen Verbindungen im Kulturmedium in nicht zytotoxischer Konzentration in 96er Microtiterplatten verdünnt. Anschließend werden Fibroblastenzellen vom Schaf ($5 \times 10^4$ Zellen pro Napf) in Produktionsmedium zu jedem Näpfchen zugeführt. Jedes Näpfchen erhält dann 50 $\mu$l einer VISNA-Viruslösung mit einem Titer von ca. $2{,}5 \times 10^4$ $TCID_{50}$ (TCID = tissue culture infectious dosis). Diese Virusdosis entspricht einer MOI (Multiplizität der Infektion) von ca. 0,05.

Unter diesen Infektionsbedingungen resultiert zwischen Tag 5 und Tag 10 in einer Infektionskontrolle ohne Substanz ein virusinduzierter, zytopathischer Effekt. Die infizierten und behandelten Zellen und die Kontrollzellen werden 7 Tage bei 37°C und 5 % $CO_2$ inkubiert.

Bei Auftreten des virusinduzierten zytopathogenen Effektes in der unbehandelten Viruskontrolle werden die Kulturen mit Formalin fixiert und anschließend mit einer Giemsa-Lösung gefärbt.

Die Wirkstärke wird in drei Stufen mit der Bedeutung "1" ca. 20 % Inhibition, "2" ca. 70 - 80 % Inhibition und "3" vollständige Inhibition der Virenvermehrung, gemessen an der Syncytien-Bildung, beurteilt.

Tabelle 7

| Wirkung gegen den VISNA-Virus | | | | | |
|---|---|---|---|---|---|
| Konz. (µl/ml) | 500 | 250 | 125 | 62,5 | 31,3 |
| Verbindung nach | | | | | |
| Beispiel 1 | 2 | 2 | 2 | 2-1 | 1 |
| Beispiel 2 | 2-3 | 2 | 2 | 2 | 2-1 |
| Beispiel 3 | 2 | 2 | 2 | 2-1 | 2-1 |
| Xylansulfat | 2 | 2 | 2 | 2 | 2 |

**4. Wirkung der substituierten Polysaccharide auf die Blutgerinnung**

Für die Bewertung des Einflusses der Substanzen auf den Gerinnungsablauf wird die Thrombinzeit (TZ) und die partielle Thrombinzeit (PTZ) bestimmt. Als Vergleichssubstanz wird Heparin herangezogen, und zwar in einer Konzentration von 6 IU/ml. Von den erfindungsgemäßen, substituierten Polysacchariden werden jeweils Konzentrationen eingesetzt, die gewichtsmäßig den 6 IU Heparin entsprechen.

Die Substanzen werden abgewogen und in einer Konzentration von 6 mg/ml in einer physiologischen Salzlösung mit 1 % Albumin gelöst. Davon werden jeweils 10 µl zu 1 ml Citratplasma gegeben. Nach 15 Min. Inkubation bei Raumtemperatur werden TZ und PTZ bestimmt.

Die Ergebnisse der Versuche sind in Tabelle 8 aufgezeichnet. Die Ergebnisse zeigen, daß die erfindungsgemäßen substituierten Polysaccharide die Blutgerinnung weitaus weniger beeinflussen als Heparin und Xylansulfat.

Tabelle 8

| Wirkung auf die Blutgerinnung | | |
|---|---|---|
| Verbindung nach | TZ (Sec.) | PTZ (Sec.) |
| Beispiel 21 | 18,1 | 61,5 |
| 22 | 19,4 | 55,2 |
| 23 | 21,7 | 72,4 |
| 24 | 24,9 | 124,3 |
| 25 | 19,5 | 67,2 |
| Xylansulfat | 28,4 | 400 |
| Heparin (6 IU/ml) | 400 | 400 |
| Kontrolle | 18,3 | 41,8 |

Allgemeine Arbeitsvorschrift I:

Alkylierung und Sulfatierung von Polysacchariden

Das Kohlenhydrat wird in wasserfreiem DMSO ca. 5 %ig gelöst und unter Ausschluß von Sauerstoff (Stickstoff oder Argon-Atmosphäre) mit den für den gewünschten Veretherungsgrad notwendigen Mengen Kaliumhydroxid und Alkylbromid oder Alkyljodid (Verhältnis Base zu Alkylierungsmittel 1,5:1) unter Erwärmung 16 Stunden gerührt. Dann gibt man einen 1,5- bis 3-fachen Überschuß an Schwefeltrioxid-Pyridin-Komplex zu (bezogen auf die freien OH-Gruppen) und sulfatiert weitere 3 Stunden bei 50 °C. Die Lösung wird anschließend im Vakuum eingeengt, der Rückstand in Wasser aufgenommen und mit wäßriger NaOH neutralisiert. Die Reinigung und Entsalzung erfolgt durch Ultrafiltration über eine geeignete Membran.

Allgemeine Arbeitsvorschrift II:

Acylierung von Polysacchariden

In eine Lösung der einzuführenden Carbonsäure in wasserfreiem DMSO wird bei 50 °C unter Rühren portionsweise die äquimolare Menge N,N'-Carbonyldiimidazol (CDI) eingetragen, wobei das Entweichen von $CO_2$ die Bildung des reaktiven Acylimidazols anzeigt. Man rührt noch eine Stunde bei 50 °C und fügt dann eine Lösung des Polysaccharids in DMSO zu, wobei die Menge entsprechend dem gewünschten Veresterungsgrad zu wählen ist. Die Reaktionsmischung wird 3 Stunden bei 50 °C belassen und anschließend im Vakuum eingeengt.

Allgemeine Arbeitsvorschrift III:

Sulfatierung von Polysacchariden

Das zu sulfatierende Polysaccharid wird in DMF aufgenommen und mit einem Überschuß Schwefeltrioxid-Pyridin-Komplex (1,5 - 3 Äquivalente pro freier OH-Gruppe) 3 Stunden bei 50 °C sulfatiert. Nach dem Abziehen des Lösungsmittels nimmt man den Rückstand in Wasser auf, neutralisiert mit NaOH und engt die Lösung zur Entfernung von Pyridin und DMF nochmal ein. Der Rückstand wird wieder in Wasser gelöst und durch Ultrafiltration über eine Membran mit geeigneter Ausschlußgrenze von Salzen und Verunreinigungen befreit. Das Retentat wird mit Phosphatpuffer pH 7, 2 Gew.-% bezogen auf die Trockensubstanz, versetzt und sprüh- oder gefriergetrocknet.

**Beispiel 1** Propanoyl-xylansulfat, Na-Salz

-X-B-Y =

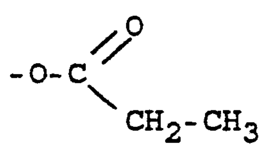

Entsprechend AAV (allgemeiner Arbeitsvorschrift) II und III werden 5 ml (4,97 g, 67 mmol) Propionsäure und 11 g (67 mmol) N,N'-Carbonyldiimidazol (CDI) in 200 ml wasserfreiem DMSO mit 15 g (114 mmol) Xylan (Molgewicht M ~ 4.000 D) umgesetzt und anschließend mit 60 g (377 mmol) $SO_3$-Pyridin-Komplex in 300 ml abs. DMF sulfatiert.

Nach Aufarbeitung, Reinigung und Gefriertrocknung erhält man 26,7 g eines gelblichen amorphen Pulvers.

Acylierungsgrad: 20 % der OH Gruppen (nach $^{13}C$-NMR in $D_2O$)
Sulfatierungsgrad: 75 % der OH-Gruppen
Analyse:
26,7 % C
4,5 % H
12,9 % S
IR (KBr): 1740 $cm^{-1}$ (sst, Ester-CO)
$^{13}C$-NMR ($D_2O$): $\delta$ = 176 - 178 (m, CO),
95 - 103 (m, C-1),
69 - 79 (m, C-2,3,4),
56 - 64 (m, C-5, CO-$\underline{C}H_2$),

28.5 (s, $CH_2$), 9.2 ppm (s, $CH_3$).

Aus dem Integral der Signale von $CH_3$ (9.2 ppm) und C-1 (95 -103 ppm) eines quantitativen $^{13}$C-NMR (INVGAT-Methode) läßt sich der Acylierungsgrad bestimmen.

**Beispiel 2** Propanoyl-dextransulfat, Na-Salz, aus Dextran 6.000 D

Entspechend AAV II und III werden 9,2 ml (9,14 g, 123 mmol) Propionsäure und 20 g (123 mmol) CDI in 200 ml wasserfreiem DMSO mit 20 g (123 mmol) Dextran (M ~ 6.000 D) umgesetzt und anschließend in 300 ml abs. DMF mit 100 g (630 mmol) $SO_3$-Pyridin-Komplex sulfatiert.

Nach Aufarbeitung, Reinigung und Trocknung erhält man 35,9 g eines weißen; amorphen Pulvers.
Acylierungsgrad: 29 % der OH-Gruppen (nach $^{13}$C-NMR)
Sulfatierungsgrad: 56 %
Analyse:
26,2 % C
4,5 % H
13,6 % S
IR (KBr): 1742 $cm^{-1}$ (sst, Ester-CO)
$^{13}$C-NMR ($D_2O$): $\delta$ = 177 (bs, CO),
95.5 - 99.5 (m, C-1),
65 - 80 (m, C-2,3,4,5,6, CO-C $H_2$),
28 (s, $CH_2$), 8.7 ppm (s, $CH_3$).

**Beispiel 3** Hexanoyl-dextransulfat, Na-Salz, aus Dextran 6.000 D

-X-B-Y = 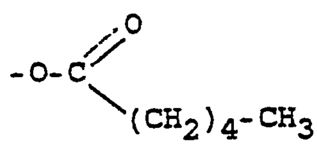

Entsprechend AAV II und III werden 14 ml (13 g, 111 mmol) Hexansäure, 18,1 g (111 mmol) CDI, 20 g (123 mmol) Dextran (M ~6.000 D) in 200 ml DMSO abs. umgesetzt, das Reaktionsprodukt anschließend mit 90 g (566 mmol) $SO_3$-Pyridin-Komplex in 300 ml DMF abs. sulfatiert.
Ausbeute: 44,2 g leicht gelbliches, amorphes Pulver
Acylierungsgrad: 20 % der OH Gruppen (nach $^{13}$C-NMR in $D_2O$)
Sulfatierungsgrad: 65 %
Analyse:
26,4 % C
4,3 % H
13,6 % S
IR (KBr): 1740 $cm^{-1}$ (sst, Ester-CO)
$^{13}$C-NMR ($D_2O$): $\delta$ = 177.5 (bs, CO),
97 - 19.5 (m, C-1),
66 - 79 (m, C-2,3,4,5,6,CO-C $H_2$),
33.5, 32.2, 25.6, 23.2 (s, 4 x $CH_2$), 5.0 ppm (s, $CH_3$).

**Beispiel 4** Dodecanoyl-dextransulfat, Na-Salz, aus Dextran 3.500 D

-X-B-Y = 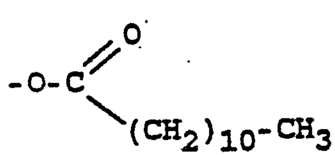

Entsprechend AAV II und III werden 14,8 g (0,074 mol) Dodecansäure, 12 g (0,074 mol) CDI, 20 g (0,123 mol) Dextran (M ~3.500) in 200 ml DMSO abs. umgesetzt und das Reaktionsprodukt mit 90 g (0,57 mol) $SO_3$-Pyridin-Komplex in 300 ml DMF abs. sulfatiert.
Ausbeute: 42,3 g weißes, amorphes Pulver
Acylierungsgrad: 6 % der OH-Gruppen (nach $^{13}C$-NMR)
Sulfatierungsgrad: 83 %
Analyse:
21,3 % C
3,1 % H
17,3 % S
IR (KBr): 1741 $cm^{-1}$ (s, Ester-CO)
$^{13}C$-NMR ($D_2O$): $\delta$ = 177.3 (bs, CO),
97 - 99 (bs, C-1),
66 - 78.5 (m, C-2,3,4,5,6, CO-C $H_2$),
35.5, 33.3, 31.4, 26.0, 24.3 (s, 9 x $CH_2$), 15.5 ppm (s, $CH_3$).

**Beispiel 5** Dodecanoyl-xylansulfat, Na-Salz

Entsprechend AAV II und III werden 9,1 g (0,045 mol) Dodecansäure, 7,36 g (0,045 mol) CDI, 15 g (0,114 mol) Xylan in 200 ml DMSO abs. umgesetzt, das Reaktionsprodukt mit 60 g (0,38 mol) $SO_3$-Pyridin-Komplex in 300 ml DMF abs. sulfatiert.
Ausbeute: 30,5 g schwach bräunliches amorphes Pulver
Acylierungsgrad: 20 % der OH-Gruppen (nach $^{13}C$-NMR)
Sulfatierungsgrad: 72.5 %
Analyse:
30,4 % C
4,4 % H
12,0 % S
IR (KBr): 1740 $cm^{-1}$ (st, Ester-CO)
$^{13}C$-NMR ($D_2O$): $\delta$ = 174 - 178.5 (bs, CO),
94.5 - 106 (bs, C-1),
57 - 83.5 (m, C-2,3,4,5, CO-C $H_2$),
35.3, 33.4, 31.5, 26.0, 24.1 (bs, 9 x $CH_2$), 15.7 ppm (s, $CH_3$).

**Beispiel 6** Hexanoyl-dextransulfat, Na-Salz, aus Dextran 10.000 D

Entsprechend AAV II und III werden 1,1 ml (1,08 g, 9 mmol) Hexansäure, 1,5 g (9 mmol) CDI, 5 g (31 mmol) Dextran (M ~10.000D) in 50 ml abs. DMSO umgesetzt, das Reaktionsprodukt mit 30 g (189 mmol) $SO_3$-Pyridin-Komplex in 100 ml abs. DMF sulfatiert.
Ausbeute: 10,7 g weißes amorphes Pulver
Acylierungsgrad: 10 % der OH-Gruppen (nach $^{13}C$-NMR)
Sulfatierungsgrad: 51 %
Analyse:
27,0 % C
4,2 % H
14,1 % S
IR (KBr): 1738 $cm^{-1}$ (m, Ester-CO)
$^{13}C$-NMR ($D_2O$): $\delta$ = 175.5 - 178 (m, CO),
96 - 99 (m, C-1),
66 - 81 (m, C=2,3,4,5,6, CO-C $H_2$),
35.7, 32.1, 25.5, 23.4 (s, 4 x $CH_2$), 15.0 ppm (s, $CH_3$).

**Beispiel 7** Hexanoyl-dextransulfat, Na-Salz, aus Dextran 10.000 D

Entsprechend AAV II und III werden 2,2 ml (2,16 g, 18 mmol) Hexansäure, 3,0 g (18 mmol) CDI, 5 g (31

mmol) Dextran (M~10.000 D) in 50 ml abs. DMSO umgesetzt, das Reaktionsprodukt mit 30 g (189 mmol) $SO_3$-Pyridin-Komplex in 100 ml DMF sulfatiert.
Ausbeute: 11,8 g weißes, amorphes Pulver
Acylierungsgrad: 20 % der OH-Gruppen (nach $^{13}C$-NMR)
Sulfatierungsgrad: 57 %
Analyse:
29,1 % C
4,3 % H
13,8 % S
IR (KBr): 1740 $cm^{-1}$ (st, Ester-CO)
$^{13}C$-NMR ($D_2O$): $\delta$ = 176 - 178.5 (bs, CO),
96.5 - 99.5 (bs, C-1),
66 - 82 (m, C-2,3,4,5,6, CO-C $H_2$),
35.6, 32.1, 25.8, 23.5, (s, 4 x $CH_2$), 15.2 ppm (s, $CH_3$).

**Beispiel 8** Hexanoyl-dextransulfat, Na-Salz, aus Dextran 10.000 D

Entsprechend AAV II und III werden 5,8 ml (5,38 g, 46 mmol) Hexansäure, 7,5 g (46 mmol) CDI, 5 g (31 mmol) Dextran (M~10.000 D) in 50 ml DMSO abs. umgesetzt, das Reaktionsprodukt anschließend mit 20 g (126 mmol) $SO_3$-Pyridin-Komplex in 100 ml abs. DMF sulfatiert.
Ausbeute: 12,2 g weißes, amorphes Pulver
Acylierungsgrad: 44 % der OH-Gruppen (nach $^{13}C$-NMR)
Sulfatierungsgrad: 50 %
Analyse:
33,6 % C
5,2 % H
9,7 % S
IR (KBr): 1741 $cm^{-1}$ (sst, Ester-CO)
$^{13}C$-NMR ($D_2O$): $\delta$ = 172.5 - 179 (m, CO),
96.2 - 99.7 (m, C-1),
65 - 79 (m, C-2,3,4,5,6, CO-C $H_2$),
35.6, 32.2, 25.7, 23.8 (s, 4 x $CH_2$), 14.8 ppm (s, $CH_3$).

**Beispiel 9** Hexadecanoyldextransulfat, Na-Salz, aus Dextran 6.000 D

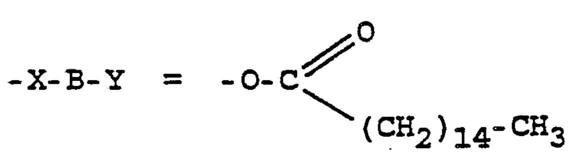

Entsprechend AAV II und III werden 1,9 g (7,4 mmol) Hexadecansäure, 1,2 g (7,4 mmol) CDI, 2 g (12,3 mmol) Dextran (M~6.000 D) in 20 ml DMSO abs. umgesetzt, das Reaktionsprodukt anschließend mit 10 g (63 mmol) $SO_3$-Pyridin-Komplex in 100 ml DMF abs. sulfatiert.
Ausbeute: 4,3 g weißes, amorphes Pulver
Acylierungsgrad: 20 % der OH-Gruppen (nach $^{13}C$-NMR)
Sulfatierungsgrad: 70 %
Analyse:
26,9 % C
4,8 % H
12,6 % S
IR (KBr): 1740 $cm^{-1}$ (st, Ester-CO),
2860, 2930 $cm^{-1}$ (st, $CH_2$)
$^{13}C$-NMR ($D_2O$): $\delta$ = 174 - 179.5 (m, CO),
98.5- 100.5 (m, C-1),

62 - 83 (m, C-2,3,4,5,6, CO-C $H_2$),
39.0, 34.9, 32.8, 25.5 (bs, 14 x $CH_2$), 16.8 ppm (s, $CH_3$).

**Beispiel 10** Dodecanoyldextransulfat, Na-Salz, aus Dextran 6.000 D

Entsprechend AAV II und III werden 15 g (75 mmol) Dodecansäure, 12 g (75 mmol) CDI, 20 g (123 mmol) Dextran (M~6.000 D) in 200 ml DMSO abs. umgesetzt, das Reaktionsprodukt mit 90 g (566 mmol) $SO_3$-Pyridin-Komplex in 300 ml DMF abs. sulfatiert.
Ausbeute: 47,4 g schwach gelbliches amorphes Pulver
Acylierungsgrad: 20 % der OH-Gruppen (nach $^{13}$C-NMR)
Sulfatierungsgrad: 48 %
IR (KBr): 1740 $cm^{-1}$ (m, Ester-CO)
$^{13}$C-NMR ($D_2O$): $\delta$ = 175.5 - 180 (m, CO),
97 - 100.5 (m, C-1),
65.5 - 79.5 (m, C-2,3,4,5,6, CO-C $H_2$),
35.6, 33.8, 31.0, 26.2, 24.0 (bs, 10 x $CH_2$), 15.5 ppm (s, $CH_3$).

**Beispiel 11** Hexanoyldextransulfat, Na-Salz, aus Dextran 6.000 D

Acylierungsgrad: 14 % der OH-Gruppen (nach $^{13}$C-NMR)
Sulfatierungsgrad: 66 %
Analyse:
22,9 % C
3,9 % H
14,1 % S

**Beispiel 12** Propanoyldextransulfat, Na-Salz, aus Dextran 6.000 D

Acylierungsgrad: 22 % der OH-Gruppen
Sulfatierungsgrad: 59 %
Analyse:
23,7 % C
4,0 % H
13,9 % S

**Beispiel 13** Hexanoylxylansulfat, Na-Salz

Acylierungsgrad: 25 % der OH-Gruppen
Sulfatierungsgrad: 70 %
Analyse:
24,5 % C
2,8 % H
13,8 % S

**Beispiel 14** [(1,3-Di-isopropoxyl)-propyl-2-oxy-carbonyl]-propanoyl-dextransulfat, Na-Salz, aus Dextran 10.000 D

-X-B-Y =

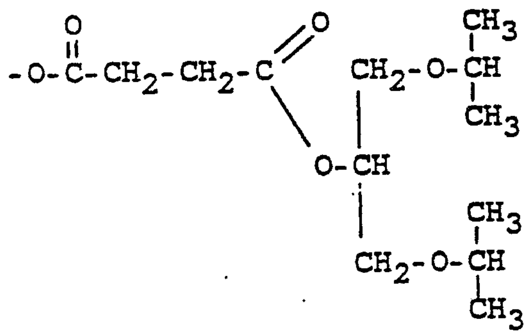

Acylierungsgrad: 10 % der OH-Gruppen
Sulfatierungsgrad: 66 %
Analyse:
25,0 % C
4,1 % H
13,3 % S

**Beispiel 15** Hexanoyl-dextransulfat, Na-Salz, aus hydriertem Dextran 3.500 D

Acylierungsgrad: 20 % der OH-Gruppen
Sulfatierungsgrad: 78 %
Analyse:
23,8 % C
3,4 % H
15,9 % S

**Beispiel 16** Propanoyldextransulfat, Na-Salz, aus Dextran 10.000 D

Acylierungsgrad: 38 % der OH-Gruppen
Sulfatierungsgrad: 53 %
Analyse:
24,7 % C
4,0 % H
11,2 % S

**Beispiel 17** Hexanoyldextransulfat, Na-Salz, aus Dextran 20.000 D

Acylierungsgrad: 8 % der OH-Gruppen
Sulfatierungsgrad: 55 %
Analyse:
27,2 % C
3,1 % H
16,0 % S

**Beispiel 18** Propanoyldextransulfat, Na-Salz, aus Dextran 3.500 D

Acylierungsgrad: 21 % der OH-Gruppen
Sulfatierungsgrad: 65 %
Analyse:
23,0 % C
4,1 % H

15,2 % S

**Beispiel 19** Dodecanoyldextransulfat, Na-Salz, aus Dextran 10.000 D

Acylierungsgrad: 17 % der OH-Gruppen
Sulfatierungsgrad: 80 %
Analyse:
25,9 % C
4,6 % H
14,6 % S

**Beispiel 20** Hexanoylxylansulfat, Na-Salz

Acylierungsgrad: 10 % der OH-Gruppen
Sulfatierungsgrad: 78 %
Analyse:
21,7 % C
3,9 % H
14,6 % S

**Beispiel 21** Propanoylxylansulfat, Na-Salz

Acylierungsgrad: 20 % der OH-Gruppen
Sulfatierungsgrad: 72 %
Analyse:
22,5 % C
3,1 % H
12,7 % S

**Beispiel 22** Propanoylxylansulfat, Na-Salz

Acylierungsgrad: 39 % der OH-Gruppen
Sulfatierungsgrad: 54 %
Analyse:
26,6 % C
3,5 % H
10,4 % S

**Beispiel 23** Hexanoylxylansulfat, Na-Salz

Acylierungsgrad: 16 % der OH-Gruppen
Sulfatierungsgrad: 56 %
Analyse:
26,65 % C
3,65 % H
11,4 % S

**Beispiel 24** Dodecanoylxylansulfat, Na-Salz

Acylierungsgrad: 12 % der OH-Gruppen
Sulfatierungsgrad: 81 %
Analyse:

24,5 % C
3,7 % H
13,4 % S

**Beispiel 25** Hexadecanoylxylansulfat, Na-Salz

20 g (152 mmol) Xylan werden in 200 ml wasserfreiem DMSO entsprechend AAV II mit 15,5 g (60.5 mmol) Palmitinsäure und 9,8 g (60.5 mmol) N,N'-Carbonyldiimidazol 3 Stunden bei 70 °C umgesetzt. Das Lösungsmittel wird anschließend im Vakuum abgezogen und der Rückstand entsprechend AAV III in 200 ml DMF mit 80 g (0.5 mol) Schwefeltrioxid-Pyridin-Komplex 3 Stunden bei 50 °C sulfatiert.

Nach Aufarbeitung, Ultrafiltration und Gefriertrocknung erhält man 46,2 g eines gelblichen, amorphen Pulvers.

Acylierungsgrad: 20 % der OH-Gruppen
Sulfatierungsgrad: 50 %
Analyse:
38,65 % C
5,95 % H
9,1 % S
IR (KBr): 1742 $cm^{-1}$ (sst, Ester-CO)
$^{13}C$-NMR ($D_2O$): $\delta$ = 171-178.5 (m, CO),
92.5-100.5 (m, C-1),
47-81 (m, C-2,3,4,5, CO-C $H_2$),
34.1, 31.8, 26.5, 24.4 (m, 14 x $CH_2$), 15.2 ppm (s, $CH_3$).

**Beispiel 26** Hexyldextransulfat, Na-Salz, aus hydriertem Dextran 6.500 D

-X-B-Y = -O-$(CH_2)_5$-$CH_3$

Entsprechend AAV I werden 5 g hydriertes Dextran (M ~ 6.500 D) in 100 ml wasserfreiem DMSO gelöst und mit 1,2 g gepulvertem Natriumhydroxid und 3,9 ml 1-Bromhexan (entsprechend 30 % eq. bezogen auf die OH-Gruppen) unter Stickstoffatmosphäre 8 Stunden bei 50 °C umgesetzt. Anschließend gibt man unter kräftigem Rühren 22 g Schwefeltrioxid-Pyridin-Komplex zu und sulfatiert 3 Stunden bei 50 °C. Nach Aufarbeitung, Ultrafiltration und Gefriertrocknung erhält man 8,9 g eines gelblichen, amorphen Pulvers.

Alkylierungsgrad: 12 % der OH-Gruppen
Sulfatierungsgrad: 83 %
Analyse:
20,1 % C
2,9 % H
16,4 % S
$^{13}C$-NMR ($D_2O$): $\delta$ = 97 - 99.5 (m, C-1),
65.5- 81 (m, C-2,3,4,5,6, O-$CH_2$),
32.4, 30.7, 26.3, 23.5 (4s, 4 x $CH_2$) 15.1 ppm (s, $CH_3$).

**Beispiel 27** Butyldextransulfat, Na-Salz, aus Dextran 10.000 D

-X-B-Y = -O-$(CH_2)_3$-$CH_3$

Entsprechend allgemeiner Arbeitsvorschrift I werden 5 g Dextran (M ~ 10.000 D) in 100 ml DMSO abs. unter Stickstoffatmosphäre mit 2 ml 1-Brombutan und 1 g gepulvertem Kaliumhydroxid 8 Stunden bei 50 °C umgesetzt. Anschließend wird 3 Stunden bei 50 °C mit 22 g Schwefeltrioxid-Pyridin-Komplex sulfatiert. Nach Aufarbeitung, Reinigung und Gefriertrocknung erhält man 8,1 g eines schwach gelblich gefärbten, amorphen Pulvers.

Alkylierungsgrad: 8 % der OH-Gruppen
Sulfatierungsgrad: 72 %
Analyse:
20,1 % C

3,1 % H
16,8 % S
$^{13}C$-NMR ($D_2O$): δ = 98.2 - 99.5 (m, C-1),
65.5 - 84.5 (m, C-2,3,4,5,6, O-$CH_2$),
33.2, 20.5 (bs, 2 x ($CH_2$), 15.3 ppm (bs, $CH_3$).

**Beispiel 28** Octyldextransulfat, Na-Salz, aus Dextran 10.000 D

-X-B-Y = -O-$(CH_2)_7$-$CH_3$

Entsprechend allgemeiner Arbeitsvorschrift I werden 5 g Dextran (M ~ 10.000 D) mit 3,4 ml 1-Jodoctan und 1 g gepulvertem NaOH unter Stickstoff umgesetzt und anschließend mit 22 g Schwefeltrioxid-Pyridin-Komplex sulfatiert.
Ausbeute: 10,2 g hellbräunliches amorphes Pulver
Alkylierungsgrad: 7 % der OH-Gruppen
Sulfatierungsgrad:89 %
Analyse:
18,7 % C
2,7 % H
17,6 % S
$^{13}C$-NMR ($D_2O$): δ = 96.5 - 99.5 (m, C-1),
67 - 78.5 (m, C-2,3,4,5,6, O-$CH_2$),
33.6, 31.0, 27.5, 24.2 (bs, 6 x $CH_2$), 15.8 ppm (s, $CH_3$).

**Beispiel 29** Hexyldextransulfat, Na-Salz, aus hydriertem Dextran 6.500 D

Entsprechend allgemeiner Arbeitsvorschrift I werden 5 g hydriertes Dextran (M ~ 6.500 D) in 100 ml DMSO abs. mit 5,2 ml 1-Bromhexan und 1,6 g gepulvertem Natriumhydroxid unter Stickstoffatmopshäre umgesetzt und anschließend mit 22 g Schwefeltrioxid-Pyridin-Komplex sulfatiert.
Ausbeute: 8,8 g beiges amorphes Pulver
Alkylierungsgrad: 7 % der OH-Gruppen
Sulfatierungsgrad:80 %
Analyse:
18,3 % C
2,9 % H
16,1 % S
$^{13}C$-NMR ($D_2O$): δ = 98.5 (bs, C-1),
67.0 - 80.5 (m, C-2,3,4,5,6; O-$CH_2$),
32.5, 30.5, 26.2, 23.8 (bs, 4 x $CH_2$), 14.9 ppm (bs, $CH_3$).

**Beispiel 30** Hexyldextransulfat, Na-Salz , aus hydriertem Dextran 10.000 D

Entsprechend allgemeiner Arbeitsvorschrift I werden 5 g hydriertes Dextran (M ~ 6.500 D) in 100 ml wasserfreiem DMSO mit 7,8 ml 1-Bromhexan und 2,4 g Natriumhydroxid unter Stickstoffatmosphäre bei 70 °C umgesetzt und anschließend mit 22 g Schwefeltrioxid-Pyridin-Komplex sulfatiert.
Ausbeute: 7,2 g eines bräunlichen, amorphen Pulvers
Alkylierungsgrad: 17 % der OH-Gruppen
Sulfatierungsgrad:64 %
Analyse:
26,7 % C
3,9 % H
15,2 % S
$^{13}C$-NMR ($D_2O$): = 97.2 - 99.5 (m, C-1),
66.0 - 80.5 (m, C-2,3,4,5,6, O-$CH_2$),
32.5, 30.7, 26.2, 23.9 (bs, 4 x $CH_2$), 15.0 ppm (s, $CH_3$).

## Ansprüche

1. Substituierte Polysaccharide, die linear oder verzweigt sein können, bestehend aus einheitlichen oder unterschiedlichen natürlichen oder synthetischen Monomeren, und pro Monomereinheit auch eine substituierte oder unsubstituierte Aminogruppen enthalten können und deren OH-Gruppen durchschnittlich zwischen 5 und 80 % substituiert sind mit einer Gruppe der Formel -X-B-Y, wobei
X eine Oxygruppe oder eine Gruppe der Formel I bedeutet

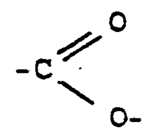

(I)

deren Kohlenstoffatom an B gebunden ist,
B einen nicht-aromatischen Kohlenwasserstoffrest mit 2 bis 30 C-Atomen bedeutet, in dessen Alkylkette bis zu 3 Methyleneinheiten durch Oxygruppen ersetzt sein können, in der bis zu drei C-C-Doppelbindungen vorliegen können und der substituiert sein kann mit bis zu drei $C_1$-$C_4$-Alkylresten und
Y - falls X eine Oxygruppe ist - Wasserstoff, COOR, $OSO_3R^1$ sein kann, worin R ein physiologisch verträgliches ein- oder zweiwertiges Kation ist, oder einen Kohlenwasserstoffrest mit bis zu 20 C-Atomen oder einen Mono- oder Bisetherrest mit 3 bis 10 C-Atomen darstellt,
$R^1$ ein physiologisch verträgliches Kation darstellt oder
Y - falls X ein Rest der Formel I ist - Wasserstoff oder COOR darstellt, worin R die obengenannten Bedeutungen hat
und wobei die OH-Gruppen der obengenannten Polysaccharide zwischen 10 und 95 % substituiert sind mit einer Gruppe der Formel $OSO_3M$, wobei M ein physiologisch verträgliches Kation darstellt und zwischen 0 und 40 % der OH-Gruppen unsubstituiert sind mit der Ausnahme der Palmitinsäureester von Chondroitinsulfat und Laurinsäureester von Dextransulfat.

2. Substituierte Polysaccharide gemäß Anspruch 1, dadurch gekennzeichnet, daß X eine Oxygruppe ist und Y Wasserstoff, COOR, $OSO_3R^1$ sein kann, worin R ein physiologisch verträgliches Kation ist oder einen Kohlenwasserstoffrest mit bis zu 20 C-Atomen darstellt und $R_1$ ein physiologisch verträgliches Kation darstellt.

3. Substituierte Polysaccharide gemäß Anspruch 1, dadurch gekennzeichnet, daß X eine Gruppe der Formel I ist und Y Wasserstoff oder COOR darstellt, wobei R die genannten Bedeutungen besitzt.

4. Substituierte Polysaccharide gemäß einem oder mehreren der Ansprüche 1 bis 3, dadurch gekennzeichnet, daß das Polysaccharidgerüst aus einheitlichen oder verschiedenen Monomereinheiten, ausgewählt aus der folgenden Gruppe von Verbindungen aufgebaut ist bzw. aus folgenden Verbindungen besteht: Xylose, Arabinose, Rhamnose, Fucose, Glucose, Mannose, Galactose, Fructose, Glucosamin, Galactosamin, Mannosamin, Glucuronsäure, Galacturonsäure, Mannuronsäure, Carragenane, Fucoidan, Laminaran, Alginat, Lentinan, Pluran, Xylan, Dextran, Heparin, Keratansulfat, Chondroitin-4- und -6-sulfat, Dermatansulfat, Heparinsulfat, Hyaluronsäure, Teichuronsäure sowie Teilhydrolysate von Stärke, Cellulose, Glycogen, Chitin oder Pektin.

5. Substituierte Polysaccharide gemäß einem oder mehreren der Ansprüche 1 - 4, dadurch gekennzeichnet, daß sie ein mittleres Molekulargewicht von bis zu 100 KD aufweisen.

6. Substituierte Polysaccharide gemäß einem oder mehreren der Ansprüche 1 bis 5, dadurch gekennzeichnet, daß das Polysaccharidgerüst aus Xylan oder Dextran mit einem mittleren Molekulargewicht von 1000 bis 22000 besteht.

7. Verfahren zur Herstellung von substituierten Polysacchariden, in denen X eine Oxygruppe ist, gemäß einem oder mehreren der Ansprüche 1, 2 oder 4 bis 6, dadurch gekennzeichnet, daß die Polysaccharide unter basischer Katalyse mit einem Alkylhalogenid oder einem $\omega$-Halogencarbonsäuresalz partiell verethert werden und anschließend direkt oder nach Alkoxylierung ganz oder teilweise sulfatiert werden.

8. Verfahren zur Herstellung von substituierten Polysacchariden, in denen X eine Gruppe der Formel I ist, gemäß einem oder mehreren der Ansprüche 1, 3 oder 4 bis 6, dadurch gekennzeichnet, daß die Polysaccharide mit Carbonsäuren oder Carbonsäurederivaten partiell acyliert werden und die verbleibenden OH-Gruppen der Polysaccharide ganz oder teilweise sulfatiert werden.

9. Verwendung von substituierten Polysacchariden gemäß einem oder mehreren der vorausgehenden Ansprüche, wobei die Palmitinsäureester von Chondroitinsulfat und die Laurinsäureester von Dextransulfat nicht ausgenommen sind, zur Bekämpfung von Krankheiten, die durch Viren hervorgerufen werden,

insbesondere von Krankheiten, die durch das HIV hervorgerufen werden.

10. Arzneimittel gekennzeichnet durch einen Gehalt an Verbindungen gemäß Anspruch 1.

11. Arzneimittel, gekennzeichnet durch einen Gehalt an Verbindungen gemäß einem oder mehreren der vorausgehenden Ansprüche zur Bekämpfung von Viruserkrankungen, insbesondere zur Bekämpfung von Krankheiten hervorgerufen durch das HIV.

12. Verwendung von Verbindungen gemäß einem oder mehreren der Ansprüche 1 bis 6 zur Herstellung von Arzneimitteln zur Bekämpfung von Viruserkrankungen, insbesondere zur Bekämpfung von Krankheiten, hervorgerufen durch das HIV.

13. Verfahren zur Behandlung von Viruserkrankungen, dadurch gekennzeichnet, daß man eine oder mehrere Verbindungen gemäß einem oder mehreren der Ansprüche 1 bis 6 verabreicht.

14. Verfahren zur Herstellung von Arzneimitteln zur Bekämpfung von Viruserkrankungen, dadurch gekennzeichnet, daß eine oder mehrere Verbindung(en) gemäß einem oder mehreren der Ansprüche 1 - 6 mit geeigneten Hilfs- und/oder Trägerstoffen in eine geeignete Darreichungsform gebracht wird.

Patentansprüche für folgende Vertragsstaaten: ES, GR

1. Verfahren zur Herstellung von substituierten Polysacchariden, die linear oder verzweigt sein können, bestehend aus einheitlichen oder unterschiedlichen natürlichen oder synthetischen Monomeren, und pro Monomereinheit auch eine substituierte oder unsubstituierte Aminogruppen enthalten können und deren OH-Gruppen durchschnittlich zwischen 5 und 80 % substituiert sind mit einer Gruppe der Formel -X-B-Y, wobei

X eine Oxygruppe oder eine Gruppe der Formel I bedeutet

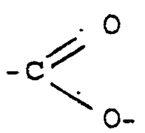

(I)

deren Kohlenstoffatom an B gebunden ist,

B einen nicht-aromatischen Kohlenwasserstoffrest mit 2 bis 30 C-Atomen bedeutet, in dessen Alkylkette bis zu 3 Methyleneinheiten durch Oxygruppen ersetzt sein können, in der bis zu drei C-C-Doppelbindungen vorliegen können und der substituiert sein kann mit bis zu drei $C_1$-$C_4$-Alkylresten und

Y - falls X eine Oxygruppe ist - Wasserstoff, COOR, $OSO_3R^1$ sein kann, worin R ein physiologisch verträgliches ein- oder zweiwertiges Kation ist, oder einen Kohlenwasserstoffrest mit bis zu 20 C-Atomen oder einen Mono- oder Bisetherrest mit 3 bis 10 C-Atomen darstellt,

$R^1$ ein physiologisch verträgliches Kation darstellt oder

Y - falls X ein Rest der Formel I ist - Wasserstoff oder COOR darstellt, worin R die obengenannten Bedeutungen hat

und wobei die OH-Gruppen der obengenannten Polysaccharide zwischen 10 und 95 % substituiert sind mit einer Gruppe der Formel $OSO_3M$, wobei M ein physiologisch verträgliches Kation darstellt und zwischen 0 und 40 % der OH-Gruppen unsubstituiert sind mit der Ausnahme der Palmitinsäureester von Chondroitinsulfat und Laurinsäureester von Dextransulfat, dadurch gekennzeichnet, daß zur Herstellung von substituierten Polysacchariden , in denen X eine Oxygruppe ist, die Polysaccharide unter basischer Katalyse mit einem Alkylhalogenid oder einem ω-Halogencarbonsäuresalz partiell verethert werden und anschließend direkt oder nach Alkoxylierung ganz oder teilweise sulfatiert werden oder zur Herstellung von substituierten Polysacchariden, in denen X eine Gruppe der Formel I ist, die Polysaccharide mit Carbonsäuren oder Carbonsäurederivaten partiell acyliert werden und die verbleibenden OH-Gruppen der Polysaccharide ganz oder teilweise sulfatiert werden.

2. Verfahren gemäß Anspruch 1, dadurch gekennzeichnet, daß X eine Oxygruppe ist und Y Wasserstoff, COOR, $OSO_3R^1$ sein kann, worin R ein physiologisch verträgliches Kation ist oder einen Kohlenwasserstoffrest mit bis zu 20 C-Atomen darstellt und $R_1$ ein physiologisch verträgliches Kation darstellt.

3. Verfahren gemäß Anspruch 1, dadurch gekennzeichnet, daß X eine Gruppe der Formel I ist und Y Wasserstoff oder COOR darstellt, wobei R die genannten Bedeutungen besitzt.

4. Verfahren gemäß einem oder mehreren der Ansprüche 1 bis 3, dadurch gekennzeichnet, daß das Polysaccharidgerüst aus einheitlichen oder verschiedenen Monomereinheiten, ausgewählt aus der folgenden Gruppe von Verbindungen aufgebaut ist bzw. aus folgenden Verbindungen besteht: Xylose, Arabinose, Rhamnose, Fucose, Glucose, Mannose, Galactose, Fructose, Glucosamin, Galactosamin, Mannosamin,

Glucuronsäure, Galacturonsäure, Mannuronsäure, Carragenane, Fucoidan, Laminaran, Alginat, Lentinan, Pluran, Xylan, Dextran, Heparin, Keratansulfat, Chondroitin-4- und -6-sulfat, Dermatansulfat, Heparinsulfat, Hyaluronsäure, Teichuronsäure sowie Teilhydrolysate von Stärke, Cellulose, Glycogen, Chitin oder Pektin.

5. Verfahren gemäß einem oder mehreren der Ansprüche 1 - 4, dadurch gekennzeichnet, daß die Polysaccharide ein mittleres Molekulargewicht von bis zu 100 KD aufweisen.

6. Verfahren gemäß einem oder mehreren der Ansprüche 1 bis 5, dadurch gekennzeichnet, daß das Polysaccharidgerüst aus Xylan oder Dextran mit einem mittleren Molekulargewicht von 1000 D bis 22000 D besteht.

7. Verwendung von substituierten Polysacchariden gemäß einem oder mehreren der vorausgehenden Ansprüche, wobei die Palmitinsäureester von Chondroitinsulfat und die Laurinsäureester von Dextransulfat nicht ausgenommen sind, zur Bekämpfung von Krankheiten, die durch Viren hervorgerufen werden, insbesondere von Krankheiten, die durch das HIV hervorgerufen werden.

8. Arzneimittel gekennzeichnet durch einen Gehalt an Verbindungen hergestellt gemäß Anspruch 1.

9. Arzneimittel, gekennzeichnet durch einen Gehalt an Verbindungen, hergestellt gemäß einem oder mehreren der vorausgehenden Ansprüche, zur Bekämpfung von Viruserkrankungen, insbesondere zur Bekämpfung von Krankheiten hervorgerufen durch das HIV.

10. Verwendung von Verbindungen, hergestellt gemäß einem oder mehreren der Ansprüche 1 bis 6, zur Herstellung von Arzneimitteln zur Bekämpfung von Viruserkrankungen, insbesondere zur Bekämpfung von Krankheiten, hervorgerufen durch das HIV.

11. Verfahren zur Behandlung von Viruserkrankungen, dadurch gekennzeichnet, daß man eine oder mehrere Verbindungen, hergestellt gemäß einem oder mehreren der Ansprüche 1 bis 6, verabreicht.

12. Verfahren zur Herstellung von Arzneimitteln zur Bekämpfung von Viruserkrankungen, dadurch gekennzeichnet, daß eine oder mehrere Verbindung(en), hergestellt gemäß einem oder mehreren der Ansprüche 1 -6 mit geeigneten Hilfs- und/oder Trägerstoffen in eine geeignete Darreichungsform gebracht wird.

Europäisches Patentamt

# EUROPÄISCHER RECHERCHENBERICHT

Nummer der Anmeldung

EP 90 11 2230

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-4 177 345 (R. SCHWEIGER)<br>* Zusammenfassung *<br>--- | 1 | C 08 B 37/00<br>C 08 B 37/02<br>C 08 B 37/14<br>A 61 K 31/72<br>A 61 K 31/725 |
| X | CHEMICAL ABSTRACTS, Band 79, Nr. 16, 22. Oktober 1973, Seite 84, Zusammenfassung Nr. 93706d, Columbus, Ohio, US; & JP-A-73 00 973 (SEIKAGAKU KOGYO) 09-01-1973 (Kat. D)<br>* Zusammenfassung *<br>--- | 1 | |
| X | CHEMICAL ABSTRACTS, Band 85, Nr. 20, 15. November 1976, Seite 120, Zusammenfassung nr. 145099d, Columbus, Ohio, US; & SU-A-334 840 (M.P. KOZLOV et al.) 25-07-1976<br>* Zusammenfassung *<br>--- | 1,8 | |
| X | US-A-3 478 016 (M. ROWLEY)<br>* Ansprüche *<br>--- | 1,7,8 | |
| A | FR-A-2 236 513 (AJINOMOTO)<br>--- | 1 | **RECHERCHIERTE SACHGEBIETE (Int. Cl.5)** |
| D,A | EP-A-0 240 098 (K.K. UENO SEIYAKU OYO KENKYUJO)<br>--- | | C 08 B<br>A 61 K |
| P,X | EP-A-0 347 907 (SOLCO BASEL)<br>* Zusammenfassung *<br>----- | 1 | |

Der vorliegende Recherchenbericht wurde für alle Patentansprüche erstellt

| Recherchenort | Abschlußdatum der Recherche | Prüfer |
|---|---|---|
| DEN HAAG | 09-10-1990 | SOMERVILLE F.M. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)